(19) Europäisches Patentamt
European Patent Office
Office européen des brevets

(11) Numéro de publication: **0 056 343**
**B1**

(12) **FASCICULE DE BREVET EUROPEEN**

(45) Date de publication du fascicule du brevet: **04.04.84**

(51) Int. Cl.[3]: **C 07 C 103/183**, C 07 C 103/127

(21) Numéro de dépôt: **82400013.7**

(22) Date de dépôt: **07.01.82**

(54) **Procédé de préparation de l'amino-4 butyramide.**

(30) Priorité: **13.01.81 FR 8100440**

(43) Date de publication de la demande: **21.07.82 Bulletin 82/29**

(45) Mention de la délivrance du brevet: **04.04.84 Bulletin 84/14**

(84) Etats contractants désignés: **AT BE CH DE FR GB IT LI LU NL SE**

(56) Documents cités:
**FR - A - 1 225 419**
**FR - A - 2 224 451**
**FR - A - 2 226 388**

(73) Titulaire: **SYNTHELABO, 58, rue de la Glacière, F-75621 Paris Cedex 13 (FR)**

(72) Inventeur: **Gosteli, Jacques, Cerecon A.G., CH-4416 Bubendorf (CH)**
Inventeur: **Mangane, Michel, 44, avenue Marcel Cachin, F-92320 Châtillon-sous-Bagneux (FR)**
Inventeur: **Rossey, Guy, 8,Square Lebrun,Voisins-le-Bretonneux, F-78180 Montigny-le-Bretonneux (FR)**
Inventeur: **Wick, Alexander, 10 Boulevard des Plantes, F-78800 St-Nom-la-Bretèche (FR)**

(74) Mandataire: **Thouret-Lemaitre, Elisabeth et al, Service Brevets - SYNTHELABO 58, rue de la Glacière, F-75621 Paris Cedex 13 (FR)**

EP 0 056 343 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Procede de preparation de l'amino-4 butyramide

La présente invention concerne un nouveau procédé de préparation du gabamide.

Dans la littérature, différentes préparations du gabamide ou amino-4 butyramide ont été déjà décrites, en particulier l'article de Angew. Chem. Int. Ed. 19 (1980), p. 627 décrit l'hydrogénation sélective du cyano-3 propionamide en présence de chlorure d'hydrogène.

La présente invention est relative à un procédé économique conduisant au gabamide avec un très bon rendement. Le procédé de l'invention est représenté dans le schéma suivant:

$$NO_2-CH_2-CH_2-CH_2-COO\ \text{alkyle} \xrightarrow{NH_4OH/HCl} NO_2-CH_2-CH_2-CH_2-CONH_2$$

$$\downarrow [H]/HCl$$

$$NH_2-CH_2-CH_2-CH_2-CONH_2$$

Le composé de départ, un nitro-4-butanoate d'alkyle plus particulièrement le nitro-4-butanoate de méthyle, est décrit dans la littérature: J. Org. Chem. 1962, 1609.

La première étape du procédé consiste à transformer le nitro-4 butanoate d'alkyle en nitro-4 butyramide par tout moyen approprié de transformation d'un ester en amide, par exemple par aminolyse, en particulier en présence d'ammoniaque et de chlorure d'ammonium. Le nitro-4 butyramide est connu et a été préparé par une méthode différente, décrite également dans J. Org. Chem. 1962, 1609. Un exemple d'aminolyse d'ester méthylique se trouve dans le brevet français n° 2 224 451.

La réduction du nitro-4 butyramide en amino-4 butyramide est effectuée par hydrogénation en présence d'un catalyseur; elle peut être effectuée, en particulier, en présence d'un acide, sous une pression allant par exemple de 1 à 10 bars et à une température allant de 0 à 50° C.

Les catalyseurs d'hydrogénation sont, par exemple, l'oxyde de platine $PtO_2$ ou le nickel de RANEY.

L'acide préféré est l'acide chlorhydrique.

Ces conditions douces sont à comparer avec celles qu'indique le brevet français n° 2 226 388 pour l'hydrogénation du nitro-6 hexanamide.

L'exemple suivant illustre l'invention.

### 1. Nitro-4 butyramide

Dans un erlenmeyer de 500 ml on introduit 15 g (0,102 m) de nitro-4 butanoate de méthyle dans 60 ml d'ammoniaque (d. 0.92) et 1,5 g (0,03 m) de chlorure d'ammonium. On agite magnétiquement pendant une nuit à la température du laboratoire. On concentre la solution sous vide à 45–50° C. On reprend le résidu par 200 ml d'acétate d'éthyle et filtre la solution de ses sels; on ajoute un peu de charbon actif, filtre et concentre la solution à un tiers du volume initial. Le produit cristallise sous forme de cristaux blancs. F = 89° C (Kofler)/88–89° C (Tottoli : 1° C/mn).

Rendement: 13,47 g (100%).

Les spectres IR et RMN sont conformes.

### 2. Amino-4 butyramide (chlorhydrate)

Dans une bombe de Parr, on introduit 0,25 g (1,9 mMole) de nitro-4 butyramide, 20 ml de méthanol, 5 ml d'acide chlorhydrique 1 N et 0,02 g de $PtO_2$. On agite pendant 2 heures à 20°C sous hydrogène (4 bars). On filtre le platine et concentre la solution. La masse résiduelle est reprise par 5 ml d'éthanol. La solution est alors additionnée de 20 ml d'acétate d'éthyle. Le produit cristallisé est filtré et séché. F = 139–140° C (Kofler); 138° C (Tottoli : 1° C/mn).

Rendement: 0,218 g (98%).

Les spectres IR et RMN sont identiques au produit authentique.

Le procédé de l'invention permet d'obtenir, avec un très bon rendement et de manière économique le gabamide, produit connu pour ses propriétés psychotropes et produit de départ pour des synthèses conduisant à des dérivés benzylidéniques ayant de précieuses propriétés pharmacologiques.

**Revendications**

1. Procédé de préparation de l'amino-4 butyramide caractérisé en ce que l'on réduit le nitro-4

butyramide par une hydrogénation en présence d'un catalyseur.

2. Procédé selon la revendication 1, caractérisé en ce que le catalyseur est l'oxyde de platine ou le nickel de Raney.

3. Procédé selon la revendication 2, caractérisé en ce que l'on effectue l'hydrogénation en présence d'oxyde de platine et d'un acide.

4. Procédé selon la revendication 3 caractérisé en ce que l'acide est l'acide chlorhydrique.

5. Procédé selon la revendication 1 caractérisé en ce que l'on obtient le nitro-4 butyramide à partir d'un nitro-4 butanoate d'alkyle.

6. Procédé selon la revendication 5, caractérisé en ce que l'on transforme un nitro-4 butanoate d'alkyle en nitro-4 butyramide par aminolyse.

7. Procédé selon la revendication 6 caractérisé en ce que l'aminolyse est effectuée à l'aide d'ammoniaque et de chlorure d'ammonium.

**Patentansprüche**

1. Verfahren zur Herstellung von 4-Aminobuttersäureamid dadurch gekennzeichnet, daß man 4-Nitrobuttersäureamid mittels einer Hydrierung in Gegenwart eines Katalysators reduziert.

2. Verfahren nach Anspruch 1, dadurch gekennzeichnet, daß der Katalysator aus Platinoxyd oder aus Raney-Nickel besteht.

3. Verfahren nach Anspruch 2, dadurch gekennzeichnet, daß man die Hydrierung in Gegenwart Platinoxyds und einer Säure vornimmt.

4. Verfahren nach Anspruch 3, dadurch gekennzeichnet, daß die Säure aus Salzsäure besteht.

5. Verfahren nach Anspruch 1, dadurch gekennzeichnet, daß man das 4-Nitro-buttersäureamid von einem 4-Nitro-buttersäure-alkylester ausgehend erhält.

6. Verfahren nach Anspruch 5, dadurch gekennzeichnet, daß man ein 4-Nitro-buttersäure-alkylester in 4-Nitro-buttersäureamid durch Aminolyse umwandelt.

7. Verfahren nach Anspruch 6, dadurch gekennzeichnet, daß man die Aminolyse mit Ammoniak und Ammoniumchlorid bewirkt.

**Claims**

1. A process for the preparation of 4-aminobutyramide characterized in that 4-nitrobutyramide is reduced by hydrogenation in the presence of a catalyst.

2. A process according to claim 1, characterized in that the catalyst is platinum oxide or Raney nickel.

3. A process according to claim 2, characterized in that the hydrogenation is carried out in the presence of platinum oxide and an acid.

4. A process according to claim 3, characterized in that the acid is hydrochloric acid.

5. A process according to claim 1, characterized in that the 4-nitrobutyramide is obtained from an alkyl 4-nitrobutanoate.

6. A process according to claim 5, characterized in that an alkyl 4-nitrobutanoate is converted to 4-nitrobutyramide by aminolysis.

7. A process according to claim 6, characterized in that the aminolysis is carried out using ammonia solution and ammonium chloride.